# EUROPEAN PATENT SPECIFICATION

(11) **EP 4 364 675 B1**
(45) Date of publication and mention of the grant of the patent: **27.08.2025**
(21) Application number: 23207111.8
(22) Date of filing: 31.10.2023
(51) Int. Cl.: A61B 17/132

(54) **TOURNIQUET WITH MULTI-ANGLE TIGHTENING DEVICE**
STAUBINDE MIT MEHRWINKEL-STRAFFVORRICHTUNG
GARROT AVEC DISPOSITIF DE SERRAGE MULTI-ANGLE

(30) Priority: 07.11.2022 TW 111142474
(43) Date of publication of application: 08.05.2024
(73) Proprietor: Huang, Chu-Hsiung, Taichung City 40758 (TW)
(72) Inventor: HUANG, Chu-Hsiung, 40758 Taichung City (TW); FAN, Hao-cheng, 40758 Taichung City (TW)
(74) Representative: Petraz, Gilberto Luigi

(56) References cited:
- US-A1- 2012 150 215
- US-A1- 2015 216 536
- US-A1- 2016 375 969
- US-A1- 2018 193 033
- US-A1- 2021 153 873
- US-A1- 2022 039 805
- US-A1- 2022 047 273

## Description

### FIELD OF INVENTION

The present application relates to a tourniquet, and particular to a tourniquet with a multi-angle tightening device.

### BACKGROUND OF INVENTION

Since ancient times, humans have found that using belts to tighten severed limbs, then coupled with sticks to tighten and pressurize, can effectively reduce blood loss.

However, most tourniquets in the prior art only have a single tightening device, for example, only one rod member, one ratchet, or one buckle, etc. Therefore, due to size difference between to-be-compressed limbs, when using the tightening device, it is difficult to ensure reliable compression, which is prone to cause insufficient pressure force.

In addition, in requirements of modern combat first aid, when personally operating a tourniquet, a person is required to complete all steps of pressurizing a limb within 15 seconds to prevent excessive blood loss. However, as mentioned above, because the tourniquets in the prior art have the aforesaid problems, when operated the tourniquet by one person, it is more difficult to complete the hemostasis steps within the time limit, causing many difficulties. Related art is disclosed in US 2022/039805 A1, US 2012/150215 A1, US 2016/375969 A1, US 2015/216536 A1, US 2018/193033 A1, US 2022/047273 A1 and US 2021/153873 A1.

### SUMMARY OF INVENTION

The invention is defined by appended claim 1. Preferred embodiments are set forth in the dependent claims. In the tourniquet with multi-angle tightening device of the present application, as the tightening device that can pressurize at multiple angles is used, the pressure force to a limb is sufficient, and efficiency of tourniquet usage is increased, so using the tourniquet is more convenient. Furthermore, in some embodiments of the present application, more than one tightening device is provided, which not only can achieve the aforesaid effects, but also has better reliability.

On the basis of the aforesaid purpose, the present application provides a tourniquet with a multi-angle tightening device, including a tourniquet main body, a first tightening device, and a fixing element. The first tightening device is rotatably disposed on the tourniquet main body and includes at least three pillar-shaped structures. The at least three pillar-shaped structures respectively extend outward from a rotation center of the first tightening device. There is a first width between any two adjacent pillar-shaped structures. The fixing element is disposed adjacent to the first tightening device and is disposed on the tourniquet main body. The first width is wider than the second width of the fixing element to avoid interference between the first tightening device and the fixing element. A distance from the rotation center of the first tightening device to the fixing element is less than an extension length of any one of the at least three pillar-shaped structures from the rotation center. The fixing element comprises at least one limit unit, a first curved portion, and a second curved portion. The at least one limit unit is disposed between the first curved portion and the second curved portion, the first curved portion forms a first groove, and the second curved portion forms a second groove, and concave directions of the first groove and the second groove are opposite. One of the pillar-shaped structures of the first tightening device is extended to the fixing element to be positioned against one of the first curved portion or the second curved portion.

Preferably, the tourniquet main body includes a first tightening strip, a second tightening strip, and a cover layer. A first portion and a second portion of the second tightening strip are connected to the first tightening strip, a third portion of the second tightening strip is movably disposed on the first tightening strip, and the cover layer covers the first tightening strip and the second tightening strip.

Preferably, the first tightening device is connected to the third portion of the second tightening strip through an opening of the cover layer.

Preferably, a first interval between the at least one limit unit and a top of a lateral wall of the first curved portion and a second interval between the at least one limit unit and a top of a lateral wall of the second curved portion are less than a thickness of the first tightening device.

Preferably, the multi-angle tightening device further includes a second tightening device. The second tightening device corresponds to the first tightening device and is disposed on the tourniquet main body, and the fixing element is disposed between the first tightening device and the second tightening device.

Preferably, a first attachment structure and a second attachment structure are respectively disposed on a bottom surface and a top surface of the tourniquet main body. After the tourniquet main body is wound around, the first attachment structure and the second attachment structure are attached to each other.

### DESCRIPTION OF DRAWINGS

FIG. 1 is a first top view of a tourniquet with a multi-angle tightening device according to one embodiment of the present application.
FIG. 2 is a schematic diagram of a first tightening device of the tourniquet with the multi-angle tightening device according to one embodiment of the present application.
FIG. 3 is a schematic diagram of a fixing element of the tourniquet with the multi-angle tightening device according to one embodiment of the present application.
FIG. 4 is a first sectional view of the tourniquet with the multi-angle tightening device according to one embodiment of the present application.
FIG.5 is a first implementation schematic diagram of the tourniquet with the multi-angle tightening device according to one embodiment of the present application.
FIG.6 is a second implementation schematic diagram of the tourniquet with the multi-angle tightening device according to one embodiment of the present application.
FIG.7 is a third implementation schematic diagram of the tourniquet with the multi-angle tightening device according to one embodiment of the present application.
FIG.8 is a second sectional view of the tourniquet with the multi-angle tightening device according to one embodiment of the present application.
FIG. 9 is a second top view of the tourniquet with the multi-angle tightening device according to one embodiment of the present application.
FIG. 10 is a third top view of the tourniquet with the multi-angle tightening device according to one embodiment of the present application, which is not included in the scope of appended claim 1.
FIG.11 is a third sectional view of the tourniquet with the multi-angle tightening device according to one embodiment of the present application.
FIG.12 is a fourth implementation schematic diagram of the tourniquet with the multi-angle tightening device according to one embodiment of the present application.
FIG.13 is a fifth implementation schematic diagram of the tourniquet with the multi-angle tightening device according to one embodiment of the present application.
FIG.14 is a sixth implementation schematic diagram of the tourniquet with the multi-angle tightening device according to one embodiment of the present application.
FIG. 15 is a fourth top view of the tourniquet with the multi-angle tightening device according to one embodiment of the present application.
FIG. 16 is a fifth top view of the tourniquet with the multi-angle tightening device according to one embodiment of the present application.
FIG. 17 is a sixth top view of the tourniquet with the multi-angle tightening device according to one embodiment of the present application, which is not included in the scope of appended claim 1.
FIG. 18 is a fourth sectional view of the tourniquet with the multi-angle tightening device according to one embodiment of the present application, which is not included in the scope of appended claim 1.
FIG. 19 is a fifth sectional view of the tourniquet with the multi-angle tightening device according to one embodiment of the present application, which is not included in the scope of appended claim 1.
FIG. 20 is a sixth sectional view of the tourniquet with the multi-angle tightening device according to one embodiment of the present application, which is not included in the scope of appended claim 1.
FIG. 21 is a seventh sectional view of the tourniquet with the multi-angle tightening device according to one embodiment of the present application, which is not included in the scope of appended claim 1.
FIG. 22 is an eighth sectional view of the tourniquet with the multi-angle tightening device according to one embodiment of the present application, which is not included in the scope of appended claim 1.
FIG. 23 is a ninth sectional view of the tourniquet with the multi-angle tightening device according to one embodiment of the present application, which is not included in the scope of appended claim 1.
FIG. 24 is a tenth sectional view of the tourniquet with the multi-angle tightening device according to one embodiment of the present application, which is not included in the scope of appended claim 1.
FIG. 25 is an eleventh sectional view of the tourniquet with the multi-angle tightening device according to one embodiment of the present application, which is not included in the scope of appended claim 1.
FIG. 26 is a twelfth sectional view of the tourniquet with the multi-angle tightening device according to one embodiment of the present application, which is not included in the scope of appended claim 1.

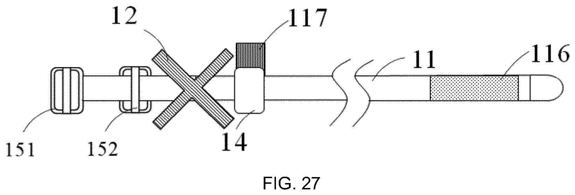

FIG. 27 is a seventh top view of the tourniquet with the multi-angle tightening device according to one embodiment of the present application.

### DETAILED DESCRIPTION OF PREFERRED EMBODIMENTS

In order to understand the technical features, content and advantages of the present application, and the effects which can achieve, the present application is hereby accompanied with the drawings and described in detail in the form of embodiments as follows. The drawings which are only for purpose of illustration and auxiliary explanation, and may not be the true proportion and precise configuration after the implementation of the present application. Therefore, it should not be interpreted based on the proportion and configuration relationship of the accompanied drawings, and limit the scope of rights of the present application in actual implementation.

The embodiments of the present application will be described below with reference to the relevant drawings. For ease of understanding, the same components in the following embodiments are described with the same symbols.

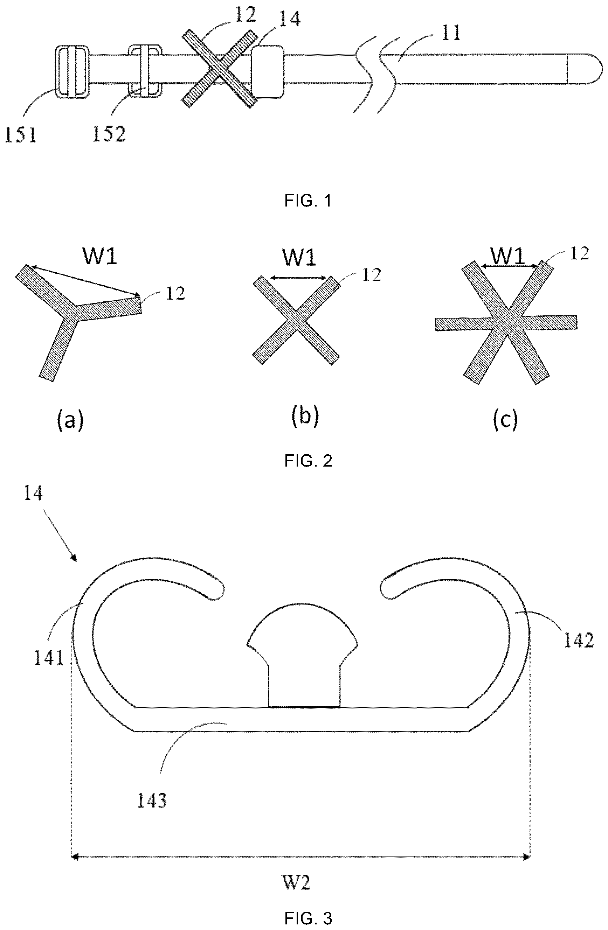

Please refer to FIG. 1 to FIG. 8, which are a first top view, first to second sectional views, first to fourth implementation schematic diagrams, a schematic diagram of a first tightening device, and a schematic diagram of a fixing element according to a tourniquet with a multi-angle tightening device of the present application.

The present application provides a tourniquet with a multi-angle tightening device, including a tourniquet main body 11, a first tightening device 12, and a fixing element 14. The first tightening device 12 is rotatably disposed on the tourniquet main body 11 and includes at least three pillar-shaped structures. The at least three pillar-shaped structures respectively extend outward from a rotation center of the first tightening device 12. There is a first width W1 between the pillar-shaped structures. The fixing element 14 is disposed adjacent to the first tightening device 12 and is disposed on the tourniquet main body 11.

As illustrated in part (a) to part (c) of FIG. 2, a number of the pillar-shaped structures can be three, four, six, etc. In detail, the number of pillar-shaped structures can also be any number such as five or more than six. Furthermore, as illustrated in FIG. 2 and FIG. 3, the first width W1 is wider than the second width W2 of the fixing element 14 to avoid interference between the first tightening device 12 and the fixing element 14. In addition, a position defined by the first width W1 depends on the length of the pillar-shaped structure and a position of the fixing element 14.

A distance from the rotation center of the first tightening device 12 to the fixing element 14 is less than an extension length of any one of the pillar-shaped structures from the rotation center. In other words, the length of the pillar-shaped structures must be ensured to be sufficiently extended to the fixing element 14 so that the fixing element 14 can fix the position of the first tightening device 12.

In one embodiment, the tourniquet main body 11 can penetrate a first buckle 151 and a second buckle 152. Therefore, the length of the tourniquet main body 11 can be adjusted, and the position of the tourniquet main body 11 can be restricted. When hemostasis is required, the tourniquet main body 11 can be wound around the limb that needs hemostasis, and then the first tightening device 12 can be used to tighten the tourniquet main body 11, so that the tourniquet main body 11 can further pressurize the limb.

More detailly, taking the first tightening device 12 including three pillar-shaped structures as an example, when rotating the first tightening device 12, at most rotating for every 120 degrees, the position of the first tightening device 12 can be fixed by the fixing element 14.

Similarly, if the first tightening device 12 includes four pillar-shaped structures, when rotating the first tightening device 12, at most rotating for every 90 degrees, the position of the first tightening device 12 can be fixed by the fixing element 14.

Similarly, if the first tightening device 12 includes six pillar-shaped structures, when rotating the first tightening device 12, at most rotating for every 60 degrees, the position of the first tightening device 12 can be fixed by the fixing element 14. Therefore, the present invention is suitable to users of various body types.

Furthermore, the fixing element 14 includes at least one limit unit 143, a first curved portion 141, and a second curved portion 142. The at least one limit unit 143 can be disposed between the first curved portion 141 and the second curved portion 142. The first curved portion 141 forms a first groove. The second curved portion 142 forms a second groove. Concave directions of the first groove and the second groove are opposite.

Therefore, if the first tightening device 12 is rotated, the second tightening strip 112 will move toward a top surface of the cover layer 114, causing the tourniquet main body 11 to be tightened and to pressurize a limb. Furthermore, in one embodiment, in order to make the first tightening device 12 be not easily damaged, the first tightening device 12 can include aluminum alloy.

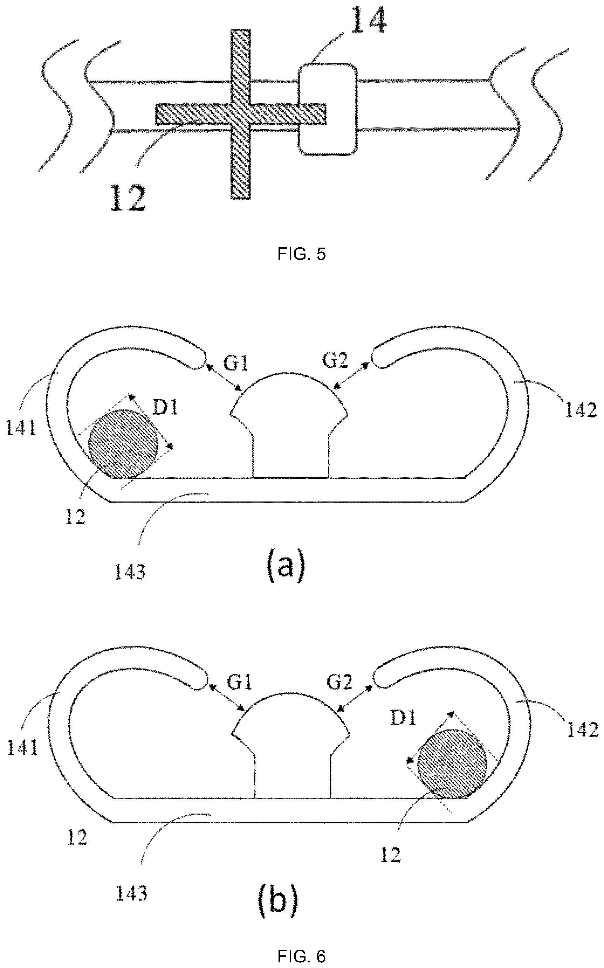

As illustrated in FIG. 5 to FIG. 7, in order to fix the first tightening device 12 and the second tightening device 13, the first tightening device 12 can be positioned against one of the first curved portion 141 or the second curved portion 142.

As illustrated in FIG. 6, in one embodiment, the limit unit 143 of the present application can be disposed between the first curved portion 141 and the second curved portion 142. For example, the limit unit 143 can be disposed at the center of the fixing element 14. The limiting unit 143 can be made of elastic material, such as polypropylene (Polypropylene, PP). A first interval G1 between the limit unit 143 and the top of the lateral wall of the first curved portion 141, and a second interval G2 between the limit unit 143 and the top of the lateral wall of the second curved portion 142 are less than a thickness D1 of the first tightening device 12 and the second tightening device 13.

Therefore, after the first tightening device 12 passes through the first curved portion 141 or the second curved portion 142, and the limit unit 143; the first tightening device 12 is restricted in the first curved portion 141 or the second curved portion 142.

Furthermore, a top of the limit unit 143 can be a three-dimensional structure with a smooth curved surface such as a hemisphere, an ellipsoid, etc., and the tops of the lateral walls of the first curved portion 141 and the second curved portion 142 can be rounded or chamfered to facilitate the first tightening device 12 and the second tightening device 13 to slide through.

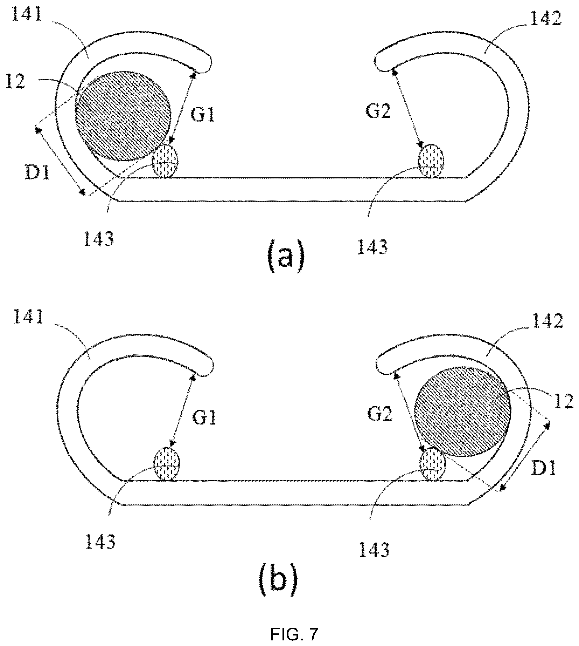

As illustrated in FIG. 7, in another embodiment, the limit unit 143 of the present application can also be disposed at another position. For example, two limit units 14 can be respectively disposed at the top of the lateral walls of the first curved portion 141 and the second curved portion 142 adjacent to the fixing element 14. The limiting units 143 can also be made of elastic material, such as polypropylene (Polypropylene, PP). The first interval G1 between the limit unit 143 and the top of the lateral wall of the first curved portion 141, and the second interval G2 between the limit unit 143 and the top of the lateral wall of the second curved portion 142 are less than the thickness D1 of the first tightening device 12 and the second tightening device 13.

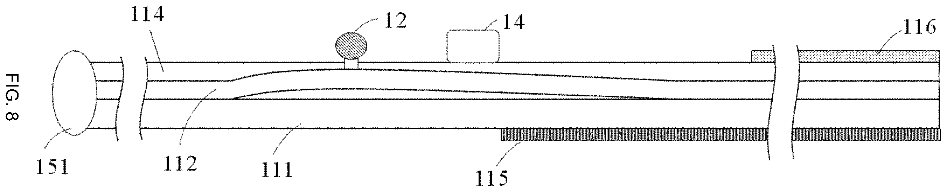

In one embodiment, as illustrated in FIG. 8, the tourniquet main body 11 includes a first tightening strip 111, a second tightening strip 112, and a cover layer 114. As illustrated in FIG. 8, a first portion and a second portion of the second tightening strip 112 are connected to the first tightening strip 111, and a third portion of the second tightening strip 112 is movably disposed on the first tightening strip 111. The cover layer 114 can cover the first tightening strip 111 and the second tightening strip 112. The first tightening device 12 is connected to the third portion of the second tightening strip 112 through an opening of the cover layer 114.

In detail, the first portion and the second portion of the second tightening strip 112 can be respectively located at two ends of the first tightening strip 111. The first portion of the second tightening strip 112 can be sewed together with the first tightening strip 111, and the second portion of the second tightening strip 112 and one end of the first tightening strip 111 can be connected to the first buckle 151.

Furthermore, in this embodiment, the first interval G1 between the limit unit 143 and the top of the lateral wall of the first curved portion 141, and the second interval G2 between the limit unit 143 and the top of the lateral wall of the second curved portion 142 are less than the thickness D1 of the first tightening device 12. Therefore, similar to the embodiment in which the limit unit 143 is disposed at the center of the fixing element 14, the first tightening device 12 can be restricted in the first curved portion 141 and the second curved portion 142.

Similarly, in this embodiment, the top of the limit unit 143 can also be a three-dimensional structure with a smooth curved surface such as a hemisphere, an ellipsoid, etc., and the tops of the lateral walls of the first curved portion 141 and the second curved portion 142 can be rounded or chamfered to facilitate the first tightening device 12 and the second tightening device 13 to slide through.

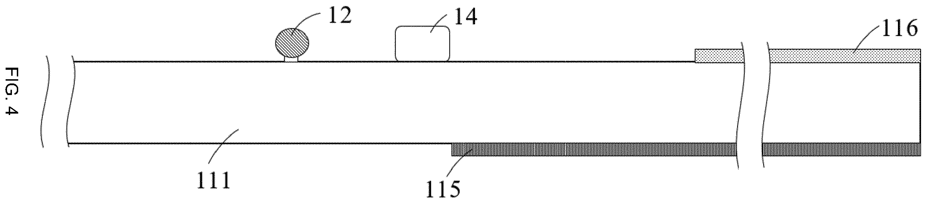

In addition to the aforesaid embodiment including the second tightening strip 112, the present application can also have other implementation forms. As illustrated in FIG. 4, the second tightening strip 112 and the third tightening strip 113 can also need not to be provided in the tourniquet with the multi-angle tightening device of the present application. In detail, the first tightening device 12 can be directly disposed on the first tightening strip 111. As illustrated in FIG. 4, when the first tightening device 12 includes pillar-shaped structures, the fixing element 14 needs to be provided. When the first tightening device 12 is a ratchet or other tightening device that can fix its own position, the fixing element 14 can be omitted.

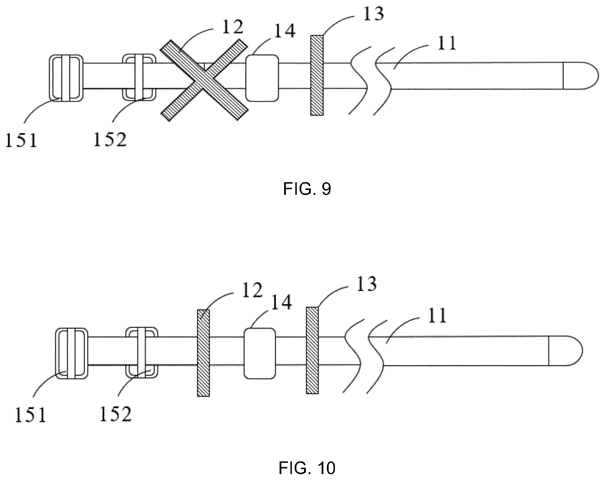

In addition to the aforesaid embodiments, as illustrated in FIG. 9 to FIG. 15, in order to realize the multi-angle tightening function, the tourniquet with the multi-angle tightening device of the present invention can further include a second tightening device 13. The second tightening device 13 can correspond to the first tightening device 12 and is disposed on the tourniquet main body 11. The fixing element 14 is disposed between the first tightening device 12 and the second tightening device 13.

In one embodiment, as illustrated in FIG. 9 to FIG. 10, as there is the second tightening device 13, the first tightening device 12 can include at least two pillar-shaped structures, which form a rod member, and the second tightening device 13 can also be a rod member.

In one embodiment, the tourniquet main body 11 can be wound around the limb which needs hemostasis, and then the tourniquet main body 11 can be tightened by the first tightening device 12, so that the tourniquet main body 11 can further pressurize the limb. After that, the first tightening device 12 is fixed in position by the fixing element 14. Besides, due to differences in the limb sizes among individuals, when the first tightening device 12 is fixed by the fixing element 14, the pressure force to the limbs may not be enough. At this time, the second tightening device 13 can be used to make the tourniquet main body 11 to be further tightened, thereby making the tourniquet main body 11 can further pressurize the limb, thus achieving the effect of multi-angle tightening.

In this embodiment, as the first tightening device 12 can also include at least three pillar-shaped structures (for example, the first tightening device 12 in FIG. 9 includes four pillar-shaped structures), the user can also only use the first tightening device 12 to pressurize the limb, and the second emergency device 13 can be used depending on requirement.

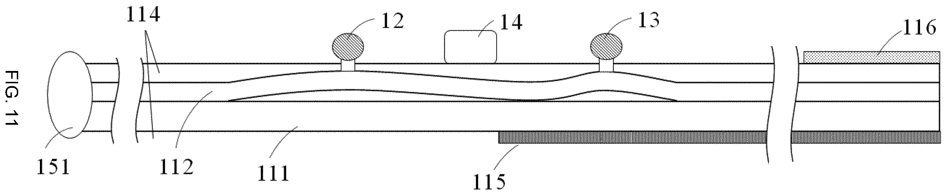

In one embodiment, the tourniquet main body 11 can include a first tightening strip 111, a second tightening strip 112, and a cover layer 114. As illustrated in FIG. 11, a first portion and a second portion of the second tightening strip 112 can be connected to the first tightening strip 111, and a third portion of the second tightening strip 112 is movably disposed on the first tightening strip 111. The cover layer 114 can cover the first tightening strip 111 and the second tightening strip 112. The first tightening device 12 and the second tightening device 13 are connected to the third portion of the second tightening strip 112 through an opening of the cover layer 114.

In detail, the first portion of the second tightening strip 112 can be sewed on the first tightening strip 111. One end of the first tightening strip 111 and the second portion of the second tightening strip 112 can be connected to the first buckle 151. Furthermore, as the third portion of the second tightening strip 112 is not sewed on the first tightening strip 111, so the third portion of the second tightening strip 112 is movably disposed on the first tightening strip 111.

Furthermore, the fixing element 14 includes at least one limit unit 143, a first curved portion 141, and a second curved portion 142. The at least one limit unit 143 can be disposed between the first curved portion 141 and the second curved portion 142. The first curved portion 141 forms a first groove. The second curved portion 142 forms a second groove. Concave directions of the first groove and the second groove are opposite.

Therefore, when the first tightening device 12 and the second tightening device 13 are rod members, if the first tightening device 12 and the second tightening device 13 are rotated, the second tightening strip 112 will move toward a top surface of the cover layer 114, causing the tourniquet main body 11 to be tightened and to pressurize a limb. Furthermore, in one embodiment, in order to make the first tightening device 12 and the second tightening device 13 be not easily damaged, the first tightening device 12 and the second tightening device 13 can include aluminum alloy.

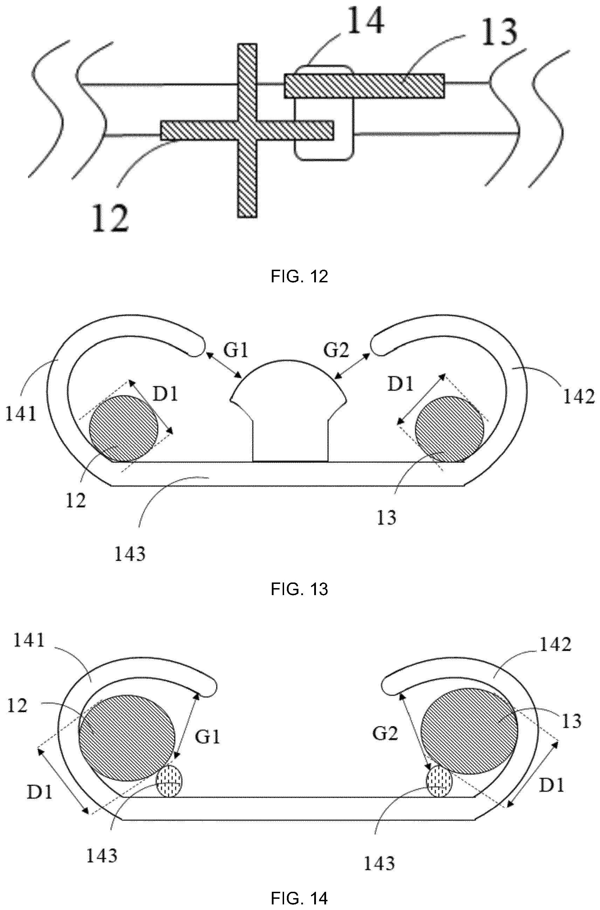

As illustrated in FIG. 13 or FIG. 14, in order to fix the first tightening device 12 and the second tightening device 13, the first tightening device 12 can be positioned against one of the first curved portion 141 or the second curved portion 142, and the second tightening device 13 can be positioned against another one of the first curved portion 141 or the second curved portion 142. In this configuration, even the pressure force of the first tightening device 12 to the first curved portion 141 or the second curved portion 142 is insufficient, the second tightening device 13 can further be used for secondary tightening to complete the process of pressing and hemostasis.

As illustrated in FIG. 13 and FIG. 14, in one embodiment, the limit unit 143 of the present application can be disposed between the first curved portion 141 and the second curved portion 142. For example, the limit unit 143 can be disposed at the center of the fixing element 14. The limiting unit 143 can be made of elastic material, such as polypropylene (Polypropylene, PP). A first interval G1 between the limit unit 143 and the top of the lateral wall of the first curved portion 141, and a second interval G2 between the limit unit 143 and the top of the lateral wall of the second curved portion 142 are less than a thickness D1 of the first tightening device 12 and the second tightening device 13.

Therefore, after the first tightening device 12 passes through the first curved portion 141 or the second curved portion 142, and the limit unit 143; the first tightening device 12 is restricted in the first curved portion 141 or the second curved portion 142. Similarly, after the second tightening device 13 passes through the first curved portion 141 or the second curved portion 142, and the limit unit 143; the second tightening device 13 is restricted in the first curved portion 141 or the second curved portion 142.

Furthermore, a top of the limit unit 143 can be a three-dimensional structure with a smooth curved surface such as a hemisphere, an ellipsoid, etc., and the tops of the lateral walls of the first curved portion 141 and the second curved portion 142 can be rounded or chamfered to facilitate the first tightening device 12 and the second tightening device 13 to slide through.

As illustrated in FIG. 14, in another embodiment, the limit unit 143 of the present application can also be disposed at another position. For example, two limit units 14 can be respectively disposed at the top of the lateral walls of the first curved portion 141 and the second curved portion 142 adjacent to the fixing element 14. The limiting units 143 can also be made of elastic material, such as polypropylene (Polypropylene, PP). The first interval G1 between the limit unit 143 and the top of the lateral wall of the first curved portion 141, and the second interval G2 between the limit unit 143 and the top of the lateral wall of the second curved portion 142 are less than the thickness D1 of the first tightening device 12 and the second tightening device 13.

Furthermore, in this embodiment, the first interval G1 between the limit unit 143 and the top of the lateral wall of the first curved portion 141, and the second interval G2 between the limit unit 143 and the top of the lateral wall of the second curved portion 142 are less than the thickness D1 of the first tightening device 12 and the second tightening device 13. Therefore, similar to the embodiment in which the limit unit 143 is disposed at the center of the fixing element 14, the first tightening device 12 and the second tightening device 13 can be restricted in the first curved portion 141 and the second curved portion 142.

Similarly, in this embodiment, the top of the limit unit 143 can also be a three-dimensional structure with a smooth curved surface such as a hemisphere, an ellipsoid, etc., and the tops of the lateral walls of the first curved portion 141 and the second curved portion 142 can be rounded or chamfered to facilitate the first tightening device 12 and the second tightening device 13 to slide through.

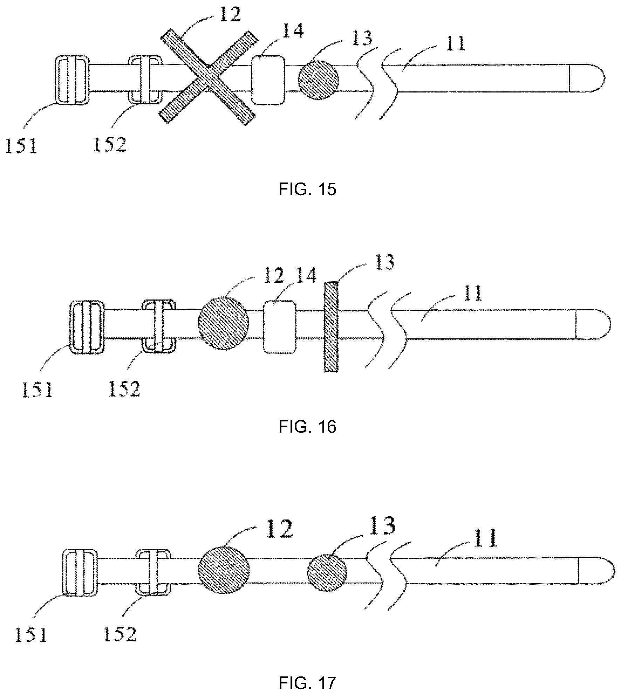

Please refer to Fig. 15 to Fig. 17, the first tightening device 12 and the second tightening device 13 can not only be the rod member including the pillar-shaped structures, but also can be in other forms. The plurality of tightening devices can make the tourniquet body 11 to pressurize the limbs multiple times.

In one embodiment, as illustrated in FIG. 15, the first tightening device 12 can be a rod member, and the second tightening device 13 can be a ratchet or other tightening device that can fix its own position. In one embodiment, as illustrated in FIG. 16, the first tightening device 12 can be a ratchet or other tightening device that can fix its own position, and the second tightening device 13 can be a rod member. In one embodiment, as illustrated in FIG. 17, the first tightening device 12 can be a ratchet or other tightening device that can fix its own position, and the second tightening device 13 can also be a ratchet or other tightening device that can fix its own position.

Different forms of the tourniquet with the multi-angle tightening device provided in FIG. 15 to FIG. 16 of the present application can also have the structures in FIG. 11 to FIG.14. For example, the tourniquet main body 11 can also include the first tightening strip 111, the second tightening strip 112, and the cover layer 114. The first curved portion 141 and the second curved portion 142 can also be formed in the fixing element 14. Furthermore, there is only one rod member in FIG. 15 and FIG. 16, so after the tourniquet main body 11 is tightened to pressurize a limb, only one rod member is restricted by the first curved portion 141 or the second curved portion 142. Related descriptions can refer to the aforesaid structures, and will not be repeated herein again.

In FIG. 17, the tourniquet with the multi-angle tightening device includes a tourniquet main body 11, a first tightening device 12, and a second tightening device 13. The first tightening device 12 can be disposed on the tourniquet main body 11. The second tightening device 13 corresponds to the first tightening device 12 and is disposed on the tourniquet main body 11.

Furthermore, the first tightening device 12 can be a ratchet or other tightening device that can fix its own position, and the second tightening device 13 can also be a ratchet or other tightening device that can fix its own position. If the first tightening device 12 and the second tightening device 13 are both ratchets or other tightening devices that can fix their own positions, there is no need to additionally dispose the fixing element 14, and the aforesaid fixing effect and the effect of pressurizing the limb with the tourniquet main body 11 multiple times can thereby being achieved.

In addition, the tourniquet with the multi-angle tightening device provided in FIG. 17 can also have the structures in FIG. 9. Specifically, the tourniquet main body 11 can also include the first tightening strip 111, the second tightening strip 112, and the cover layer 114. Furthermore, there is no rod member in the structures disclosed in FIG. 17, so no fixing element 14 is required to be further disposed.

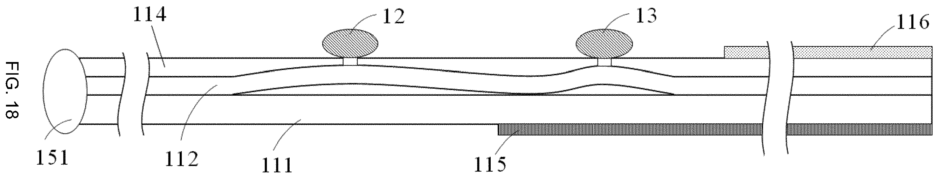

In one embodiment, as illustrated in FIG. 17 and FIG. 9, the tourniquet main body 11 can include a first tightening strip 111, a second tightening strip 112, and a cover layer 114. As illustrated in FIG. 18, a first portion and a second portion of the second tightening strip 112 can be connected to the first tightening strip 111, and a third portion of the second tightening strip 112 is movably disposed on the first tightening strip 111. The cover layer 114 can cover the first tightening strip 111 and the second tightening strip 112. The first tightening device 12 and the second tightening device 13 are connected to the third portion of the second tightening strip 112 through an opening of the cover layer 114.

In detail, the first portion and the second portion of the second tightening strip 112 can be sewed on the first tightening strip 111. Furthermore, as the third portion of the second tightening strip 112 is not sewed on the first tightening strip 111, so the third portion of the second tightening strip 112 is movably disposed on the first tightening strip 111.

Therefore, when the first tightening device 12 and the second tightening device 13 are rod members, if the first tightening device 12 and the second tightening device 13 are rotated, the second tightening strip 112 will move toward a top surface of the cover layer 114, causing the tourniquet main body 11 to be tightened and to pressurize a limb.

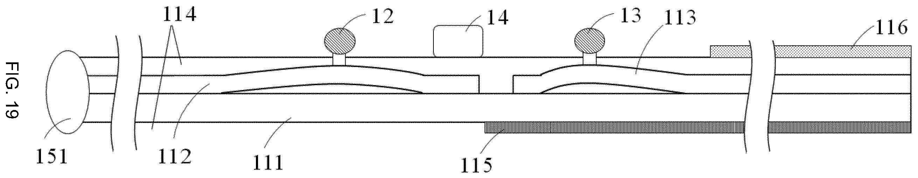

In addition to the aforesaid disclosed structures, in one embodiment, as illustrated in FIG. 19 to FIG. 24, the tourniquet main body 11 can include a third tightening strip 113. A length of the third pressure strip 113 is shorter than that of the second tightening strip 112. Two ends of the third tightening strip 113 are connected to the first tightening strip 111, the second tightening strip 112, or the cover layer 114. The first tightening device 12 is connected to the third portion of the second tightening strip 112 through the opening of the cover layer 114, and the second tightening device 13 is connected to the third tightening strip 113.

In one embodiment, as illustrated in FIG. 9, FIG. 10, FIG. 12, FIG. 15 to FIG. 16, and FIG. 19, at least one first tightening device 12 or second tightening device 13 is a rod member. The tourniquet main body 11 can include a first tightening strip 111, a second tightening strip112, a third tightening strip 113, and a cover layer 114.

As illustrated in FIG. 19, a first portion and a second portion of the second tightening strip 112 can be connected to the first tightening strip 111, and a third portion of the second tightening strip 112 is movably disposed on the first tightening strip 111. A first portion and a second portion of the third tightening strip 113 can be connected to the first tightening strip 111, and a third portion of the third tightening strip 113 is movably disposed on the first tightening strip 111. Both the second tightening strip 112 and the third tightening strip 113 can be sewed on the first tightening strip 111. The cover layer 114 can cover the first tightening strip 111, the second tightening strip 112, and the third tightening strip 113. The first tightening device 12 and the second tightening device 13 are connected to the second tightening strip 112 and the third portion of the third tightening strip 113 through the opening of the cover layer 114.

Additionally, regarding the relationship of the first tightening device 12, the second tightening device 13, and the fixing element 14, reference may be made to the aforesaid embodiments. In this embodiment, if the first tightening device 12 and the second tightening device 13 are rotated, the second tightening strip 112 can move toward the top surface of the cover layer 114, and the third tightening strip 113 can move toward the top surface of the cover layer 114, causing the tourniquet main body 11 to be tightened and to pressurize a limb.

In one embodiment, as illustrated in FIG. 9, FIG. 10, FIG. 12, FIG. 15 to FIG. 16, and FIG. 20, at least one first tightening device 12 or second tightening device 13 is a rod member. The tourniquet main body 11 can include a first tightening strip 111, a second tightening strip112, a third tightening strip 113, and a cover layer 114.

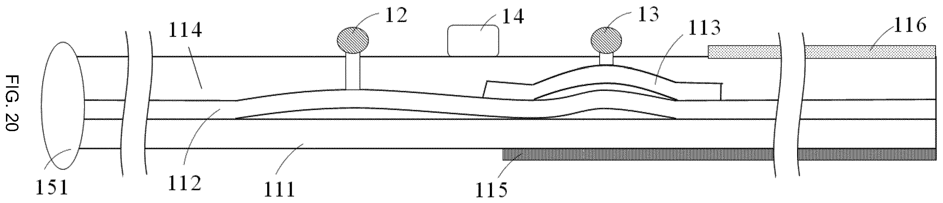

As illustrated in FIG. 20, a first portion and a second portion of the second tightening strip 112 can be connected to the first tightening strip 111, and a third portion of the second tightening strip 112 is movably disposed on the first tightening strip 111. A first portion and a second portion of the third tightening strip 113 can be connected to the second tightening strip 112, and a third portion of the third tightening strip 113 is movably disposed on the second tightening strip 112. The second tightening strip 112 can be sewed on the first tightening strip 111. The third tightening strip 113 can be sewed on the first tightening strip 111. The cover layer 114 can cover the first tightening strip 111, the second tightening strip 112, and the third tightening strip 113. The first tightening device 12 and the second tightening device 13 are connected to the second tightening strip 112 and the third portion of the third tightening strip 113 through the opening of the cover layer 114.

Additionally, regarding the relationship of the first tightening device 12, the second tightening device 13, and the fixing element 14, reference may be made to the aforesaid embodiments. In this embodiment, if the first tightening device 12 and the second tightening device 13 are rotated, the second tightening strip 112 can move toward the top surface of the cover layer 114, and the third tightening strip 113 can move toward the top surface of the cover layer 114, causing the tourniquet main body 11 to be tightened and to pressurize a limb.

In one embodiment, as illustrated in FIG. 9, FIG. 10, FIG. 12, FIG. 15 to FIG. 16, and FIG. 21, at least one first tightening device 12 or second tightening device 13 is a rod member. Another one of the first tightening device 12 or second tightening device 13 is a ratchet or other tightening device that can fix its own position. The tourniquet main body 11 can include a first tightening strip 111, a second tightening strip112, a third tightening strip 113, and a cover layer 114.

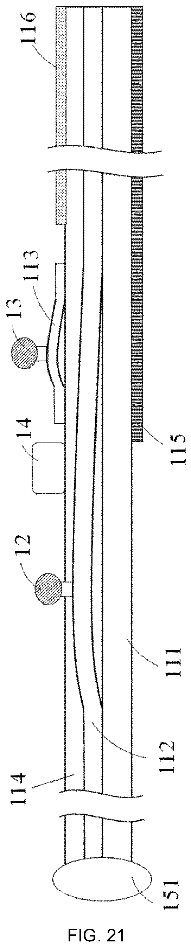

As illustrated in FIG. 21, a first portion and a second portion of the second tightening strip 112 can be connected to the first tightening strip 111, and a third portion of the second tightening strip 112 is movably disposed on the first tightening strip 111. A first portion and a second portion of the third tightening strip 113 can be connected to the cover layer 114, and a third portion of the third tightening strip 113 is movably disposed on the cover layer 114. The second tightening strip 112 can be sewed on the first tightening strip 111. The third tightening strip 113 can be sewed on the cover layer 114. The cover layer 114 can cover the first tightening strip 111 and the second tightening strip 112. The first tightening device 12 is connected to the second tightening strip 112 and the third portion of the third tightening strip 113 through the opening of the cover layer 114.

Additionally, regarding the relationship of the first tightening device 12, the second tightening device 13, and the fixing element 14, reference may be made to the aforesaid embodiments. In this embodiment, if the first tightening device 12 and the second tightening device 13 are rotated, the second tightening strip 112 can move toward the top surface of the cover layer 114, and the third tightening strip 113 can roll up part of the cover layer 114, causing the tourniquet main body 11 to be tightened and to pressurize a limb.

In one embodiment, as illustrated in FIG. 17, and FIG. 22 to FIG. 24, the first tightening device 12 can be a ratchet or other tightening device that can fix its own position, and the second tightening device 13 can also be a ratchet or other tightening device that can fix its own position. The tourniquet main body 11 can include a first tightening strip 111, a second tightening strip112, a third tightening strip 113, and a cover layer 114.

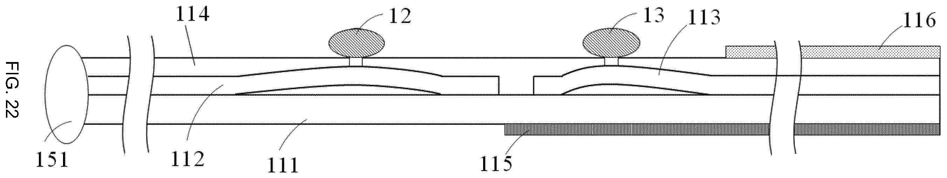

As illustrated in FIG. 22, a first portion and a second portion of the second tightening strip 112 can be connected to the first tightening strip 111, and a third portion of the second tightening strip 112 is movably disposed on the first tightening strip 111. A first portion and a second portion of the third tightening strip 113 can be connected to the first tightening strip 111, and a third portion of the third tightening strip 113 is movably disposed on the first tightening strip 111. Both the second tightening strip 112 and the third tightening strip 113 can be sewed on the first tightening strip 111. The cover layer 114 can cover the first tightening strip 111, the second tightening strip 112, and the third tightening strip 113. The first tightening device 12 and the second tightening device 13 are connected to the second tightening strip 112 and the third portion of the third tightening strip 113 through the opening of the cover layer 114.

Additionally, in this embodiment, as the first tightening device 12 and the second tightening device 13 can be ratchets or other tightening devices that can fix their own positions, no fixing element 14 is additionally disposed, and related descriptions can refer to the aforesaid embodiments. In this embodiment, if the first tightening device 12 and the second tightening device 13 are rotated, the second tightening strip 112 can move toward the top surface of the cover layer 114, and the third tightening strip 113 can move toward the top surface of the cover layer 114, causing the tourniquet main body 11 to be tightened and to pressurize a limb.

In one embodiment, as illustrated in FIG. 17, and FIG. 22 to FIG. 24, the first tightening device 12 can be a ratchet or other tightening device that can fix its own position, and the second tightening device 13 can also be a ratchet or other tightening device that can fix its own position. The tourniquet main body 11 can include a first tightening strip 111, a second tightening strip112, a third tightening strip 113, and a cover layer 114.

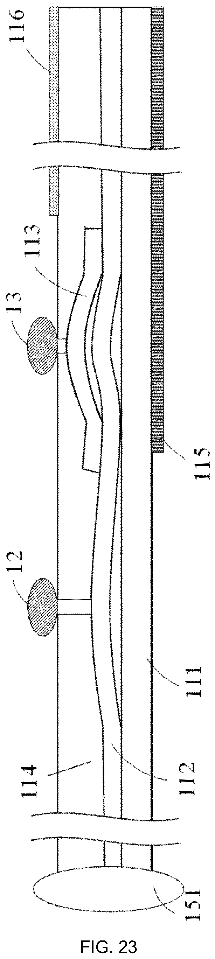

As illustrated in FIG. 23, a first portion and a second portion of the second tightening strip 112 can be connected to the first tightening strip 111, and a third portion of the second tightening strip 112 is movably disposed on the first tightening strip 111. A first portion and a second portion of the third tightening strip 113 can be connected to the second tightening strip 112, and a third portion of the third tightening strip 113 is movably disposed on the second tightening strip 112. The second tightening strip 112 can be sewed on the first tightening strip 111. The third tightening strip 113 can be sewed on the first tightening strip 111. The cover layer 114 can cover the first tightening strip 111, the second tightening strip 112, and the third tightening strip 113. The first tightening device 12 and the second tightening device 13 are connected to the second tightening strip 112 and the third portion of the third tightening strip 113 through the opening of the cover layer 114.

Additionally, in this embodiment, as the first tightening device 12 and the second tightening device 13 can be ratchets or other tightening devices that can fix their own positions, no fixing element 14 is additionally added, and related descriptions can refer to the aforesaid embodiments. In this embodiment, if the first tightening device 12 and the second tightening device 13 are rotated, the second tightening strip 112 can move toward the top surface of the cover layer 114, and the third tightening strip 113 can move toward the top surface of the cover layer 114, causing the tourniquet main body 11 to be tightened and to pressurize a limb.

In one embodiment, as illustrated in FIG. 17, and FIG. 22 to FIG. 24, the first tightening device 12 can be a ratchet or other tightening device that can fix its own position, and the second tightening device 13 can also be a ratchet or other tightening device that can fix its own position. The tourniquet main body 11 can include a first tightening strip 111, a second tightening strip112, a third tightening strip 113, and a cover layer 114.

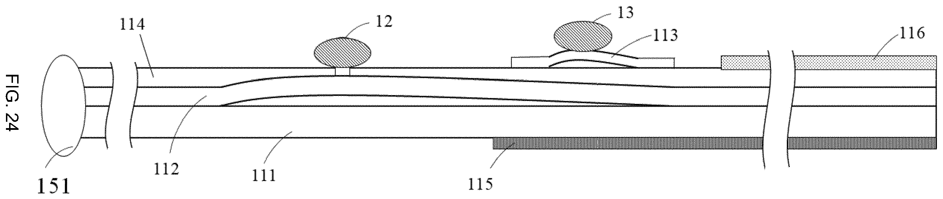

As illustrated in FIG. 24, a first portion and a second portion of the second tightening strip 112 can be connected to the first tightening strip 111, and a third portion of the second tightening strip 112 is movably disposed on the first tightening strip 111. A first portion and a second portion of the third tightening strip 113 can be connected to the cover layer 114, and a third portion of the third tightening strip 113 is movably disposed on the cover layer 114. The second tightening strip 112 can be sewed on the first tightening strip 111. The third tightening strip 113 can be sewed on the cover layer 114. The cover layer 114 can cover the first tightening strip 111 and the second tightening strip 112. The first tightening device 12 is connected to the second tightening strip 112 and the third portion of the third tightening strip 113 through the opening of the cover layer 114.

Additionally, in this embodiment, no fixing element 14 is additionally disposed, and related descriptions can refer to the aforesaid embodiments. In this embodiment, if the first tightening device 12 and the second tightening device 13 are rotated, the second tightening strip 112 can move toward the top surface of the cover layer 114, and the third tightening strip 113 can roll up part of the cover layer 114, causing the tourniquet main body 11 to be tightened and to pressurize a limb.

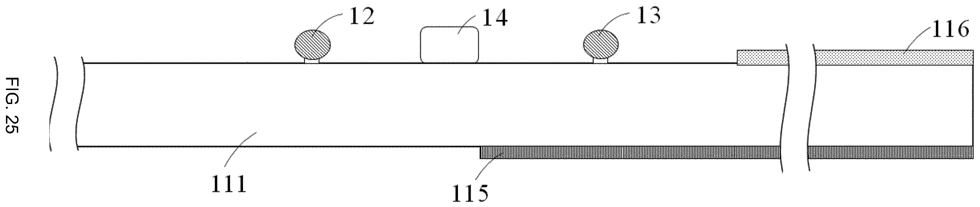

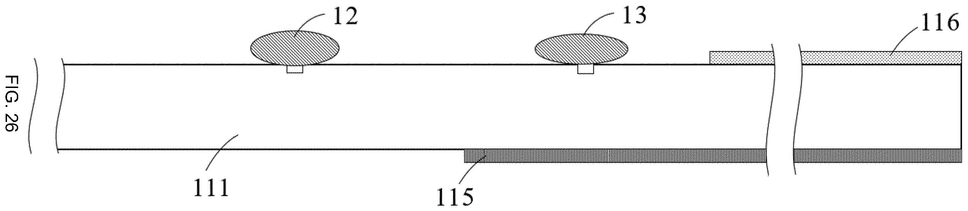

In addition to the aforesaid embodiment including the second tightening strip 112 and the third tightening strip 113, the present application can also have other implementation forms. As illustrated in FIG. 25 and FIG. 26, the second tightening strip 112 and the third tightening strip 113 can also need not to be provided in the tourniquet with the multi-angle tightening device of the present application. In detail, the first tightening device 12 and the second tightening device 13 can be sewed on the first tightening strip 111. Furthermore, the first tightening device 12 and the second tightening device 13 can be rod members, ratchets or other tightening devices that can fix their own positions. When at least one of the first tightening device 12 and the second tightening device 13 is the rod member, as illustrated in FIG. 16, the fixing element 14 needs to be provided. When the first tightening device 12 is a ratchet or other tightening device that can fix its own position, and the second tightening device 13 can also be a ratchet or other tightening device that can fix its own position, and the fixing element 14 may not be disposed.

Furthermore, as illustrated in FIG. 4, FIG. 8, FIG. 11, and FIG. 18 to FIG. 26., the first attachment structure 115 and the second attachment structure 116 can be respectively disposed on a bottom surface and a top surface of the tourniquet main body 11. Therefore, after the tourniquet main body 11 is wound around, the first attachment structure 115 and the second attachment structure 116 are attached to each other.

In one embodiment, the first attachment structure 115 and the second attachment structure 116 can include Velcro or other re-attachable elements. Therefore, after a user easily wind the tourniquet main body 11 up, the user can attach the first attachment structure 115 and the second attachment structure 116 to each other at first, and then uses the first tightening device 12 and the second tightening device 13 to tighten the tourniquet main body 11 to pressurize the limb. In this embodiment, using the first attachment structure 115 and the second attachment structure 116 can make the tourniquet main body 11 be more convenient for usage, thereby reducing emergency time.

Additionally, in one embodiment, please refer to FIG. 17, in addition to the aforesaid structures, the tourniquet with multi-angle tightening device of the present application can also include a third attachment structure 117. The third attachment structure 117 can be connected to the tourniquet main body 11. The third attachment structure 117 is a strip structure, and one surface of the third attachment structure 117 is an attachment surface, which can be a Velcro or other re-attachable elements.

After the tourniquet main body 11 is wound around, the third attachment structure 117 can be attached to the first attachment structure 115 or the second attachment structure 116. Another surface of the third attachment structure 117 is a surface for writing. The time that the tourniquet body 11 has being wound around can be written by a user on the writing surface of the third adhesive structure 117, so as to avoid secondary injury caused by excessive pressurized time to a limb.

In one embodiment, one or both of the aforesaid first attachment structure 115 or the second attachment structure 116 can be omitted, and only the third attachment structure 117 is used to fix the tourniquet main body 11 after winding.

In summary, the tourniquet with the multi-angle tightening device of the present application has various implementation forms. In the tourniquet with the multi-angle tightening device of the present application, as the limb can be pressurized at multiple angles, the problem that different limb sizes cannot be reliably pressurized for hemostasis can be solved. The present application can increase the efficiency of using the tourniquet so that the tourniquet can be more convenient to use. Furthermore, as there is more than one tightening device, the tourniquet can still be used even any one of the tightening devices being failure, so the tourniquet with the multi-angle tightening device of the present application has better reliability.

The above are only examples, but are not for restriction.

## Claims

1. A tourniquet with a multi-angle tightening device, comprising:
a tourniquet main body (11);
a first tightening device (12), rotatably disposed on the tourniquet main body (11), and comprising at least three pillar-shaped structures, wherein the at least three pillar-shaped structures respectively extend outward from a rotation center of the first tightening device (12), and there is a first width (W1) between any two adjacent pillar-shaped structures; and
a fixing element (14), disposed adjacent to the first tightening device (12), and disposed on the tourniquet main body (11),
wherein the first width (W1) is wider than a second width of the fixing element (14) to avoid interference between the first tightening device (12) and the fixing element (14), a distance from the rotation center of the first tightening device (12) to the fixing element (14) is less than an extension length of any one of the at least three pillar-shaped structures from the rotation center; wherein the tourniquet with a multi-angle tightening device is **characterized in that**
the fixing element (14) comprises at least one limit unit (143), a first curved portion (141), and a second curved portion (142); the at least one limit unit (143) is disposed between the first curved portion (141) and the second curved portion (142), the first curved portion (141) forms a first groove, and the second curved portion (142) forms a second groove, and concave directions of the first groove and the second groove are opposite; and
the length of the pillar-shaped structures of the first tightening device (12) extends to the fixing element (14) to be positioned against one of the first curved portion (141) or the second curved portion (142) so that the fixing element (14) can fix the position of the first tightening device (12).

2. The tourniquet with the multi-angle tightening device as claimed in claim 1, **characterized in that** the tourniquet main body (11) comprises a first tightening strip (111), a second tightening strip (112), and a cover layer (114); a first portion and a second portion of the second tightening strip (112) are connected to the first tightening strip (111), a third portion of the second tightening strip (112) is movably disposed on the first tightening strip (111), and the cover layer (114) covers the first tightening strip (111) and the second tightening strip (112).

3. The tourniquet with the multi-angle tightening device as claimed in claim 2, **characterized in that** the first tightening device (12) is connected to the third portion of the second tightening strip (112) through an opening of the cover layer (114).

4. The tourniquet with the multi-angle tightening device as claimed in claim 1, **characterized in that** a first interval (G1) between the at least one limit unit (143) and a top of a lateral wall of the first curved portion (141), and a second interval (G2) between the at least one limit unit (143) and a top of a lateral wall of the second curved portion (142) are less than a thickness of the first tightening device (12).

5. The tourniquet with the multi-angle tightening device as claimed in claim 1, comprising a second tightening device (13), **characterized in that** the second tightening device (13) corresponds to the first tightening device (12) and is disposed on the tourniquet main body (11), and the fixing element (14) is disposed between the first tightening device (12) and the second tightening device (13).

6. The tourniquet with the multi-angle tightening device as claimed in claim 1, **characterized in that** a first attachment structure (115) and a second attachment structure (116) are respectively disposed on a bottom surface and a top surface of the tourniquet main body (11); after the tourniquet main body (11) is wound around the limb that needs hemostasis, the first attachment structure (115) and the second attachment structure (116) are attached to each other.

7. The tourniquet with the multi-angle tightening device as claimed in claim 6, **characterized in that** the tourniquet with the multi-angle tightening device comprises a third attachment structure (117), the third attachment structure (117) is a strip structure, one surface of the third attachment structure (117) is an attachment surface, another surface of the third attachment structure (117) is a surface for writing, the third attachment structure (117) is connected to the tourniquet main body (11), after the tourniquet main body (11) is wound around the limb that needs hemostasis,
the third attachment structure (117) is attached to the first attachment structure (115) or the second attachment structure (116).

## Patentansprüche

1. Staubinde mit einer Mehrwinkel-Straffvorrichtung, umfassend:
einen Staubindehauptkörper (11);
eine erste Straffvorrichtung (12), die am Staubindehauptkörper (11) drehbar angeordnet ist, und umfassend zumindest drei säulenförmige Strukturen, worin die zumindest drei säulenförmigen Strukturen sich jeweils nach außen aus einem Drehzentrum der ersten Straffvorrichtung (12) erstrecken, und es eine erste Breite (W1) zwischen je zwei beliebigen angrenzenden säulenförmigen Strukturen gibt; und
ein Fixierelement (14), das an die erste Straffvorrichtung (12) angrenzend angeordnet ist, und am Staubindehauptkörper (11) angeordnet ist,
worin die erste Breite (W1) breiter ist als eine zweite Breite des Fixierelementes (14), um eine Störung zwischen der ersten Straffvorrichtung (12) und dem Fixierelement (14) zu vermeiden, ein Abstand vom Drehzentrum der ersten Straffvorrichtung (12) zum Fixierelement (14) kleiner ist als eine Ausdehnungslänge von irgendeiner der zumindest drei säulenförmigen Strukturen von dem Drehzentrum; worin die Staubinde mit einer Mehrwinkel-Straffvorrichtung **dadurch gekennzeichnet ist, dass**
das Fixierelement (14) zumindest eine Begrenzungseinheit (143), einen ersten gekrümmten Abschnitt (141) und einen zweiten gekrümmten Abschnitt (142) umfasst; die zumindest eine Begrenzungseinheit (143) zwischen dem ersten gekrümmten Abschnitt (141) und dem zweiten gekrümmten Abschnitt (142) angeordnet ist, der erste gekrümmte Abschnitt (141) eine erste Nut bildet, und der zweite gekrümmte Abschnitt (142) eine zweite Nut bildet, und konkave Richtungen der ersten Nut und der zweiten Nut entgegengesetzt sind; und
die Länge der säulenförmigen Strukturen der ersten Straffvorrichtung (12) sich zum Fixierelement (14) hin erstreckt, um gegen einen von dem ersten gekrümmten Abschnitt (141) oder dem zweiten gekrümmten Abschnitt (142) positioniert zu werden, so dass das Fixierelement (14) die Position der ersten Straffvorrichtung (12) fixieren kann.

2. Staubinde mit Mehrwinkel-Straffvorrichtung nach Anspruch 1, **dadurch gekennzeichnet, dass** der Staubindehauptkörper (11) einen ersten Straffstreifen (111), einen zweiten Straffstreifen (112) und eine Deckschicht (114) umfasst; ein erster Abschnitt und ein zweiter Abschnitt des zweiten Straffstreifens (112) mit dem ersten Straffstreifen (111) verbunden sind, ein dritter Abschnitt des zweiten Straffstreifens (112) am ersten Straffstreifen (111) bewegbar angeordnet ist, und die Deckschicht (114) den ersten Straffstreifen (111) und den zweiten Straffstreifen (112) deckt.

3. Staubinde mit Mehrwinkel-Straffvorrichtung nach Anspruch 2, **dadurch gekennzeichnet, dass** die erste Straffvorrichtung (12) mit dem dritten Abschnitt des zweiten Straffstreifens (112) durch eine Öffnung der Deckschicht (114) verbunden ist.

4. Staubinde mit Mehrwinkel-Straffvorrichtung nach Anspruch 1, **dadurch gekennzeichnet, dass** ein erstes Intervall (G1) zwischen der zumindest einen Begrenzungseinheit (143) und einem Oberteil einer Seitenwand des ersten gekrümmten Abschnitts (141), und ein zweites Intervall (G2) zwischen der zumindest einen Begrenzungseinheit (143) und einem Oberteil einer Seitenwand des zweiten gekrümmten Abschnitts (142) kleiner sind als eine Dicke der ersten Straffvorrichtung (12).

5. Staubinde mit Mehrwinkel-Straffvorrichtung nach Anspruch 1, umfassend eine zweite Straffvorrichtung (13), **dadurch gekennzeichnet, dass** die zweite Straffvorrichtung (13) der ersten Straffvorrichtung (12) entspricht und am Staubindehauptkörper (11) angeordnet ist, und das Fixierelement (14) zwischen der ersten Straffvorrichtung (12) und der zweiten Straffvorrichtung (13) angeordnet ist.

6. Staubinde mit Mehrwinkel-Straffvorrichtung nach Anspruch 1, **dadurch gekennzeichnet, dass** eine erste Anbringungsstruktur (115) und eine zweite Anbringungsstruktur (116) jeweils an einer Bodenfläche und einer oberen Fläche des Staubindehauptkörpers (11) angeordnet sind; nachdem der Staubindehauptkörper (11) um das Körperglied, das Hämostase benötigt, gewunden ist, die erste Anbringungsstruktur (115) und die zweite Anbringungsstruktur (116) aneinander angebracht sind.

7. Staubinde mit Mehrwinkel-Straffvorrichtung nach Anspruch 6, **dadurch gekennzeichnet, dass** die Staubinde mit der Mehrwinkel-Straffvorrichtung eine dritte Anbringungsstruktur (117) umfasst, die dritte Anbringungsstruktur (117) eine Streifenstruktur ist, eine Fläche der dritten Anbringungsstruktur (117) eine Anbringungsfläche ist, eine andere Fläche der dritten Anbringungsstruktur (117) eine Fläche zum Schreiben ist, die dritte Anbringungsstruktur (117) mit dem Staubindehauptkörper (11) verbunden ist, nachdem der Staubindehauptkörper (11) um das Körperglied, das Hämostase benötigt, gewunden ist, die dritte Anbringungsstruktur (117) an der ersten Anbringungsstruktur (115) oder der zweiten Anbringungsstruktur (116) angebracht ist.

## Revendications

1. Garrot avec un dispositif de serrage multi-angle, comprenant :
un corps principal de garrot (11) ;
un premier dispositif de serrage (12), disposé de manière rotative sur le corps principal de garrot (11), et comprenant au moins trois structures en forme de piliers, dans lequel les au moins trois structures en forme de piliers s'étendent respectivement vers l'extérieur à partir d'un centre de rotation du premier dispositif de serrage (12), et une première largeur (W1) existe entre deux structures en forme de piliers adjacentes quelconques ; et
un élément de fixation (14), disposé adjacent au premier dispositif de serrage (12), et disposé sur le corps principal de garrot (11),
dans lequel la première largeur (W1) est plus large qu'une seconde largeur de l'élément de fixation (14) pour éviter toute interférence entre le premier dispositif de serrage (12) et l'élément de fixation (14), une distance entre le centre de rotation du premier dispositif de serrage (12) et l'élément de fixation (14) est inférieure à une longueur d'extension de l'une quelconque des au moins trois structures en forme de pilier par rapport au centre de rotation ; dans lequel le garrot avec un dispositif de serrage multi-angle est **caractérisé en ce que**
l'élément de fixation (14) comprend au moins une unité de limite (143), une première partie incurvée (141) et une seconde partie incurvée (142) ; l'au moins une unité de limite (143) est disposée entre la première partie incurvée (141) et la seconde partie incurvée (142), la première partie incurvée (141) forme une première rainure, et la seconde partie incurvée (142) forme une seconde rainure, et les directions concaves de la première rainure et de la seconde rainure sont opposées ; et
la longueur des structures en forme de piliers du premier dispositif de serrage (12) s'étend jusqu'à l'élément de fixation (14) pour être positionnée contre l'une de la première partie incurvée (141) ou de la seconde partie incurvée (142) de sorte que l'élément de fixation (14) peut fixer la position du premier dispositif de serrage (12).

2. Garrot avec le dispositif de serrage multi-angle selon la revendication 1, **caractérisé en ce que** le corps principal de garrot (11) comprend une première bande de serrage (111), une seconde bande de serrage (112) et une couche de couverture (114) ; une première partie et une deuxième partie de la seconde bande de serrage (112) sont reliées à la première bande de serrage (111), une troisième partie de la seconde bande de serrage (112) est disposée de manière mobile sur la première bande de serrage (111), et la couche de couverture (114) recouvre la première bande de serrage (111) et la seconde bande de serrage (112).

3. Garrot avec le dispositif de serrage multi-angle selon la revendication 2, **caractérisé en ce que** le premier dispositif de serrage (12) est relié à la troisième partie de la seconde bande de serrage (112) à travers une ouverture de la couche de couverture (114).

4. Garrot avec le dispositif de serrage multi-angle selon la revendication 1, **caractérisé en ce qu'**un premier intervalle (G1) entre l'au moins une unité de limite (143) et un sommet d'une paroi latérale de la première partie incurvée (141), et un second intervalle (G2) entre l'au moins une unité de limite (143) et un sommet d'une paroi latérale de la seconde partie incurvée (142) sont inférieurs à une épaisseur du premier dispositif de serrage (12).

5. Garrot avec le dispositif de serrage multi-angle selon la revendication 1, comprenant un second dispositif de serrage (13), **caractérisé en ce que** le second dispositif de serrage (13) correspond au premier dispositif de serrage (12) et est disposé sur le corps principal de garrot (11), et l'élément de fixation (14) est disposé entre le premier dispositif de serrage (12) et le second dispositif de serrage (13).

6. Garrot avec le dispositif de serrage multi-angle selon la revendication 1, **caractérisé en ce qu'**une première structure de fixation (115) et une deuxième structure de fixation (116) sont respectivement disposées sur une surface inférieure et une surface supérieure du corps principal de garrot (11) ; après que le corps principal de garrot (11) soit enroulé autour du membre qui a besoin d'hémostase, la première structure de fixation (115) et la deuxième structure de fixation (116) sont fixées l'une à l'autre.

7. Garrot avec le dispositif de serrage multi-angle selon la revendication 6, **caractérisé en ce que** le garrot avec le dispositif de serrage multi-angle comprend une troisième structure de fixation (117), la troisième structure de fixation (117) est une structure en bande, une surface de la troisième structure de fixation (117) est une surface de fixation, une autre surface de la troisième structure de fixation (117) est une surface pour l'écriture, la troisième structure de fixation (117) est reliée au corps principal de garrot (11), après que le corps principal de garrot (11) soit enroulé autour du membre qui a besoin d'hémostase, la troisième structure de fixation (117) est fixée à la première structure de fixation (115) ou à la deuxième structure de fixation (116).
